# EUROPÄISCHE PATENTSCHRIFT

(11) **EP 0 399 391 B1**
(45) Veröffentlichungstag und Bekanntmachung des Hinweises auf die Patenterteilung: **13.09.1995**
(21) Anmeldenummer: 90109431.8
(22) Anmeldetag: 18.05.1990
(51) Int. Cl.: C12Q 1/00, G01N 33/52

(54) **Stabilisiertes Reagenzgemisch**
Stabilized reagent mixture
Mélange stabilisé de réactifs

(30) Priorität: 26.05.1989 DE 3917070
(43) Veröffentlichungstag der Anmeldung: 28.11.1990
(73) Patentinhaber: BOEHRINGER MANNHEIM GMBH, 68298 Mannheim (DE)
(72) Erfinder: Rothe, Anselm, Dr., D-6943 Birkenau (DE); Knappe, Wolfgang-Reinhold, Dr., D-6700 Ludwigshafen, (DE); Eikmeier, Heino, Dr., D-6143 Lorsch (DE)

(56) Entgegenhaltungen:
- EP-A- 0 016 387
- JP-A-62 074 285
- WPIL, FILE SUPPLIER, Derwent Publications Ltd., London (GB); AN 90-071181 (10)#
- WPIL, FILE SUPPLIER, Derwent Publications Ltd., London (GB); AN 87-133258 (19)#
- WPIL, FILE SUPPLIER, Derwent Publications Ltd., London (GB); AN 87-104906 (15)#
- WPI, FILE SUPPLIER, Derwent Publications Ltd., London (GB); AN 80-18504C (16)#

## Beschreibung

Gegenstand der Erfindung ist ein stabilisiertes Reagenzgemisch, welches ein oder mehrere kationenabhängige, durch redoxaktive mehrwertige Kationen inhibierbare Enzyme und ein Salz eines Komplexbildners enthält, ein Verfahren zur Herstellung dieses Reagenzgemisches und die Verwendung dieses Reagenzgemisches zur Bestimmung eines Analyten.

Die Erfindung betrifft das Gebiet der Biochemie und der Klinischen Chemie. Auf beiden Gebieten werden Reagenzgemische verwendet, die Enzyme enthalten. So werden Enzymlösungen oder Suspensionen beispielsweise zur Durchführung und Untersuchung von enzymkatalysierten Reaktionen in der Enzymologie eingesetzt. Im allgemeinen werden solche Lösungen in einem Vorratsbehälter aufbewahrt und es wird bei Bedarf aus diesem ein Teil entnommen. Diese Lösungen sollen daher über einen längeren Zeitraum stabil sein.

In der Klinischen Chemie werden Reagenzgemische, die Enzyme enthalten, besonders zur Bestimmung von Analyten beispielsweise in Körperflüssigkeiten verwendet. Hier finden neben Lösungen auch in vermehrtem Maße Trockenreagenzien Einsatz. So haben Teststreifen, auf welche die zur Bestimmung erforderlichen Reagenzien in Form eines Filmes oder Überzugs auf einen saugfähigen Träger aufgebracht sind, den Vorteil, daß sie besonders einfach in der Handhabung sind und schnell Ergebnisse erhalten werden können. Auch diese Reagenzgemische werden zweckmäßigerweise im Vorrat gehalten, so daß im Notfall schnell eine Untersuchung durchgeführt werden kann. Es ist daher erforderlich, daß die Reagenzien über längere Zeit lagerstabil sind.

In der japanischen Patentanmeldung JP-A-62074285 wurden eine Reihe von Zusätzen zur Stabilisierung des kationenunabhängigen Enzyms Monomethylaminoxidase empfohlen. Darunter werden auch chelatisierende Agenzien oder Erdalkalimetalle genannt. Die kombinierte Zugabe dieser Agenzien in einer bestimmten Reihenfolge und bestimmten Mengenverhältnissen wird nicht beschrieben.

Die Aktivität der Enzyme, welche in einem Reagenz enthalten sind, wird oft durch Schwermetallionen stark beeinträchtigt, da diese unkontrollierbare Redoxreaktionen katalysieren. In der EP-A-0 130 708 wird daher vorgeschlagen, dem Reagenzgemisch einen Komplexbildner wie Ethylendiamintetraessigsäure (EDTA) zuzusetzen.

Dadurch kann die schädigende Wirkung von Schwermetallionen auf die Enzymaktivität verringert werden. Eingesetzt wurden daher EDTA und ein Natriumsalz.

Es hat sich jedoch herausgestellt, daß der Zusatz von EDTA oder seinen Salzen zu einer praktisch vollständigen Inaktivierung von Enzymen führen kann. In der EP-B-0 075 293 wird sogar ein Verfahren zur Inhibierung von β-Galactosidase beschrieben, welches genau diese Eigenschaften ausnutzt.

Weiterhin hat es sich erwiesen, daß die Lagerstabilität der bekannten, Enzyme enthaltenden Reagenzgemische für die Zwecke der Klinischen Chemie nicht ausreichend ist.

Es bestand daher ein Bedarf an Reagenzgemischen, die mindestens ein kationenabhängiges, durch mehrwertige redoxaktive Kationen inhibierbares Enzym, ein Kation sowie einen Komplexbildner enthalten, die insbesondere die oben genannten Nachteile nicht aufweisen, jedoch die schädigende Wirkung von Schwermetallionen verhindern und eine erhöhte Lagerstabilität gewährleisten.

Es war die Aufgabe der vorliegenden Erfindung, diesen Bedarf zu befriedigen.

Diese Aufgabe wird gelöst durch das erfindungsgemäße Verfahren zur Herstellung eines stabilisierten, aktiven Reagenzgemisches, welches ein kationenabhängiges Enzym enthält, das durch redoxaktive mehrwertige Kationen inhibiert wird, das dadurch gekennzeichnet ist, daß man das kationenabhängige Enzym mit einem Salz eines Komplexbildners, das ein mehrwertiges nicht-redoxaktives Kation enthält oder mit einem Komplexbildner oder ein Salz davon zusammen mit einem Salz eines mehrwertigen, nicht-redoxaktiven Kations vermischt,
wobei die Menge des mehrwertigen nicht-redoxaktiven Kations größer oder gleich der Menge des Komplexbildners ist und die Menge des Komplexbildners größer als die Menge der anwesenden redoxaktiven Kationen ist.

Ferner umfaßt die Erfindung ein stabilisiertes Reagenzgemisch hergestellt nach diesem Verfahren, ein entsprechendes Trockenreagenzgemisch und die Verwendung des Reagenzgemisches zur Bestimmung eines Analyten.

Bei dem Enzym handelt es sich um ein Enzym, das durch Schwermetallionen, auch wenn sie in Spuren vorhanden sind, inhibiert wird. Das erfindungsgemäße Verfahren und Reagens hat dann den Vorteil, daß die Enzymaktivität trotz Anwesenheit von Schwermetallionen erhalten bleibt. Weiterhin handelt es sich uni solche Enzyme deren Aktivität von der Anwesenheit bestimmter mehrwertiger, nicht redoxaktiver Metallkationen abhängt. Stehen dem Enzym diese Kationen nicht zur Verfügung, so ist die Enzymaktivität nicht optimal. Solche Enzyme sind bekannt. Beispiele sind Hydrolasen wie β-Galaktosidase, Amylase, Cholesterinesterase, Luciferase, Proteasen sowie DNA-spaltende Enzyme oder redoxaktive Enzyme. Bei diesen Enzymen hat das erfindungsgemäße Reagenz den Vorteil, daß die Enzymaktivität nicht wie erwartet in Anwesenheit eines Komplexbildners vollständig verschwindet, sondern erhalten bleibt.

Das Reagensgemisch der Erfindung enthält einen Komplexbildner oder ein Salz eines Komplexbildners. Als Komplexbildner sind alle solchen chemischen Verbindungen zu verstehen, die mit einem oder mehreren störenden Kationen einen stabilen Komplex bilden können.

Bevorzugt sind solche Komplexbildner, die mit Schwermetallionen Komplexe bilden können. Schwermetallionen sind solche, die insbesondere die Enzymaktivität eines Enzyms hemmen oder in sonstiger Weise, z.B. durch Katalyse von unerwünschten Redoxreaktionen von Reagensbestandteilen, wie Farbabstufern, mit anderen Reagensbestandteilen, Bestandteilen einer damit in Kontakt gebrachten Probe oder des sie umgebenden Mediums, beispielsweise Luft, den bestimmungsgemäßen Gebrauch des Reagenzes beeinträchtigen. Besonders störende Ionen sind redoxaktive Ionen wie Fe³⁺, Mn²⁺ oder Co²⁺. Diese Ionen können als lösliche Verunreinigung der einzelnen Reagensbestandteile eingeschleppt worden sein. Es hat sich herausgestellt, daß viele aus der Natur isolierte Reagenzbestandteile noch störende Ionen enthalten; beispielsweise gehören dazu mineralische Trägerstoffe wie Kieselgel oder Titandioxid.

Komplexbildner der angesprochenen Art können mehrzähnige Liganden, Kryptanden, Kronenether, Podanden etc. sein. Als besonders vorteilhaft haben sich mehrzähnige Liganden und davon Nitriloessigsäuren, insbesondere Nitrilotriessigsäure und Alkylendiaminessigsäuren, insbesondere Ethylendiamintetraessigsäure (EDTA), erwiesen.

Wenn ein Salz eines Komplexbildners eingesetzt werden soll, so sind als kationischer Partner des Komplexbildners alle Kationen einsetzbar, die von dem Komplexbildner weniger stark komplexiert werden können als das störende Kation. Bevorzugt sind Kationen, die gleich stark oder weniger stark komplexiert werden als das mehrwertige, nicht redoxaktive Kation des erfindungsgemäßen Reagenzes. Auch das mehrwertige, nicht redoxaktive Kation selbst kann verwendet werden. Es sind auch Salze des Komplexbildners einsetzbar, die zwei oder mehr dieser Kationen, gleich oder verschieden, enthalten.

Bevorzugt ist der Fall, daß das Salz des Komplexbildners das mehrwertige, nicht redoxaktive Kation als kationischen Partner enthält. Dieses Kation muß dann dem Reagenz nicht mehr zusätzlich zugegeben werden, um die erfindungsgemäße Wirkung zu erzielen. Eine Zugabe ist jedoch möglich.

Falls das Salz des Komplexbildners das mehrwertige, nicht redoxaktive Kation nicht als kationischen Partner enthält, so muß dieses Kation als zusätzlicher Bestandteil, beispielsweise in Form eines Salzes, z.B. eines Halogenids, zusammen mit den Komplexbildnern dem Enzym beigefügt werden.

Die Konzentration des Komplexbildners oder seines Salzes im erfindungsgemäßen Verfahren und Reagenz hängt von der Konzentration der störenden Ionen im Reagenz ab. Sie ist daher mindestens so hoch, daß diese Ionen im wesentlichen vollständig komplexiert werden können. Die Menge des eingesetzten Komplexbildners kann daher in einfacher Weise aus der Konzentration der störenden Ionen im Reagenz ermittelt werden.

Das mehrwertige, nicht redoxaktive Kation ist ein Kation, welches die Aktivität des Enzyms im Reagenz nicht inhibiert. Bevorzugt sind Kationen, die von dem Komplexbildner weniger stark komplexiert werden als die störenden Ionen. Ganz besonders bevorzugt sind solche Kationen, die für die Enzymaktivität erforderlich sind oder sie erhöhen. Dies sind insbesondere die zweiwertigen Erdalkalionen. Mehrwertige, nicht redoxaktive Kationen, die dazu geeignet sind, die Aktivität von bestimmten Enzymen sicherzustellen, sind bekannt. Für β-Galaktosidase, Amylase, Luciferase und Cholesterinesterase ist das Kation Magnesium²⁺. Für seltenere Enzyme sind auch Calcium²⁺ bzw. Zink²⁺ verwendbar.

Der Begriff nicht redoxaktiv soll bevorzugt dahingehend verstanden werden, daß die entsprechenden Kationen Nebenreaktionen, die auf Redoxvorgängen beruhen, wie beispielsweise die Oxidation anderer Reagenzbestandteile, nicht in unbeabsichtigter Weise katalysieren.

Die Konzentration des mehrwertigen, nicht-redoxaktiven Kations orientiert sich an der Konzentration der störenden Ionen. Die Konzentration des Kations ist mindestens äquivalent zu der Menge der störenden Ionen, d.h. größer oder gleich der Konzentration des Komplexbildners, besonders bevorzugt gleich groß.

Im Fall von sogenannten Trockentests hat es sich als günstig erwiesen, alle für die Analyse eines Bestandteils einer flüssigen Probe erforderlichen Reagenzien in einen Film auf einem Teststreifen, wie beispielsweise in der DE-A-2910134 beschrieben, zu inkorporieren. Die erfindungsgemäßen Reagenzgemische sind vielseitig verwendbar. Insbesondere sind sie in vorteilhafter Weise zum Nachweis von Analyten in flüssigen Probenlösungen geeignet. Sie enthalten dann bevorzugt die für eine Nachweisreaktion oder Reaktionsfolge erforderlichen Zusatzstoffe oder werden kurz vor dem Ablaufen der Reaktion mit diesen vermischt.

Sie weisen gegenüber den bekannten Enzyme und Komplexbildner enthaltenden Reagenzmischungen den Vorteil auf, daß Störungen durch unerwünscht anwesende Ionen, beispielsweise Schwermetallionen, ausgeschlossen sind, und daß dennoch die Enzymaktivität durch den Zusatz des Komplexbildners nicht zerstört wird. Das hat beispielsweise auch zur Folge, daß die Reagenzbestandteile nicht in aufwendigen Verfahren von Schwermetallionen gereinigt werden müssen. Es können sogar weniger gut aufgearbeitete Reagenzbestandteile verwendet werden. Dies ist insbesondere bei Mineralien oder Enzymen von großem Vorteil.

In einer bevorzugten Ausführungsform der Erfindung ist ein Reagenz zum Nachweis von Cholesterin in Form eines Filmes auf einen Teststreifen aufgebracht. Das Reagens enthält die für die Führung der Reaktion erforderlichen Substanzen Tetramethylbenzidin als Farbindikator, Cholesterinoxidase, Cholesterinesterase und Peroxidase als Enzyme, und pH-Puffer sowie als Zusatzstoffe Polyvinylpropionat, Kieselgur und Gallussäure (zur Farbabstufung).

Falls ein Reagenzgemisch in Form eines Films auf einem festen Träger hergestellt werden soll, hat es sich als zweckmäßig erwiesen, zunächst eine Suspension oder Lösung aller Komponenten des Reagenzgemischs herzustellen, diese anschließend auf den Träger aufzurakeln und zu trocknen. Die Konzentration des Komplexbildners bzw. des Kations in der Reagenzmasse ist dann bevorzugt 50-500 mg/100 g Masse, oder 250-2500 mg/100 g Kieselgur, wenn gewöhnliches Kieselgur als Filmöffner benutzt wird.

Das erfindungsgemäße Reagenzgemisch hat den Vorteil, daß es sehr stabil ist. Beispielsweise verringern sich die Aktivität von Cholesterinesterase und die Konzentration von redoxempfindlichen Zusatzstoffen, wie Gallussäure, besonders in flüssigen Reagenzmassen zur Bereitung von offenen Filmen zum Nachweis von Cholesterin kaum, während in den bekannten Reagenzgemischen entweder die Enzymaktivität zerstört wird oder die Konzentration von Gallussäure absinkt oder beide Effekte beobachtet werden.

Die Stabilität des Reagenzgemisches ist enorm wichtig, da die Reagenzgemische während der Verarbeitung längere Zeit in flüssigem Zustand verbleiben müssen, bis sie in eine geeignete Form gebracht werden können.

Ein weiterer Gegenstand der Erfindung ist ein Teststreifen, der das erfindungsgemäße trockene Reagenz als Film auf einem Trägermaterial enthält.

Ebenfalls Gegenstand der Erfindung ist die Verwendung des erfindungsgemäßen Reagenzgemisches zur Bestimmung eines Analyten. Dazu wird das Reagenzgemisch eventuell vermischt mit zusätzlich erforderlichen Reagenzien mit der zu analysierenden Probe in Kontakt gebracht und die ablaufende Bestimmungsreaktion beobachtet. Beispielsweise hat sich ein Reagensgemisch das Cholesterinesterase, Cholesterinoxidase, Mg-EDTA, Gallussäure, Kieselgur, Peroxidase und Tetramethylbenzidin sowie weitere nützliche Zusatzstoffe enthält, als besonders vorteilhaft zur Bestimmung von Cholesterin in Blut erwiesen.

Folgende Zeichnungen erläutern die Erfindung:
- Abbildung 1:: Konzentrationsverlauf von Cholesterinesterase (◇), Tetramethylbenzidin ( □ ) und Gallussäure (+) in einem Reagenzgemisch nach Beispiel 1.
- Abbildung 2:: Konzentrationsverlauf von Cholesterinesterase ( ◇ ), Tetramethylbenzidin ( □ ) und Gallussäure (+) in einem Reagenzgemisch nach Beispiel 2a.
- Abbildung 3:: Konzentrationsverlauf von Cholesterinesterase ( ◇ ), Tetramethylbenzidin ( □ ) und Gallussäure (+) in einem Reagenzgemisch nach Beispiel 2b.

In den Abbildungen ist jeweils die Konzentration des Bestandteils relativ zum Anfangswert (100%) (Ordinate) gegen den Zeitpunkt der Probennahme (in Stunden) (Abszisse) aufgetragen.

Die Erfindung wird durch die folgenden Beispiele naher erlautert:

### Beispiel 1

### Herstellung eines erfindungsgemäßen Reagenzgemischs

Analog zu DE-A-2910134 wurden folgende Bestandteile gemischt:
20 g Polyvinylpropionat-Dispersion (Bindemittel, BASF, Ludwigshafen, BRD)
20 g Kieselgur (Eagle-Pichler-Industries, USA) (Celatom)
10 g Kelzan-Lösung, 1%ig (Quellmittel, Alginate Industries Inc., Hamburg, BRD)
0.5 g Tetramethylbenzidin, TMB (Boehringer Mannheim, BRD)
1.0 g Dioctylnatriumsulfosuccinat, DONS (Sigma-Chemie, Deisenhofen, BRD)
0.25 g Gallussäure-Lösung, 2.5%ig in Methanol (Merck, Darmstadt, BRD) (Farbabstufung)
10 KU Cholesterin-Oxidase EC 1.1.3.6. (Boehringer Mannheim, BRD)
40 KU Cholesterin-Esterase EC 3.1.1.13 (Boehringer Mannheim, BRD)
250 KU Peroxidase EC 1.11.1.7 (Boehringer Mannheim, BRD)
20 g Phosphatpuffer, pH 6.4
10 g H₂0, dest.
5 g Tetrahydrofuran p.A. (Merck, Darmstadt, BRD)
100 mg K₂Mg EDTA (2.4 mmol, Merck AG Darmstadt, BRD)
Die entstandene Masse wird im folgenden Masse I genannt.

### Beispiel 2

### Vergleich des Konzentrationsverlaufs der Komponenten von Reaktionsmischungen

a) Masse II
   Analog zu Beispiel 1 wurde eine Masse hergestellt, in der das K₂Mg EDTA durch eine äquivalente Menge Na₂H₂ EDTA (90 mg, 2.4 mmol, Merck AG, Darmstadt, BRD) ersetzt ist.
b) Masse III
   Analog zu Beispiel I wurde eine Masse hergestellt, jedoch die Zugabe von K₂Mg EDTA unterlassen.

In den Massen I, II und III wurde der Konzentrationsverlauf von Gallussäure und von Tetramethylbenzidin (TMB) bzw. der Aktivitätsverlauf der Cholesterinesterase (CHE) verfolgt.

Die Bestimmung der Enzyme erfolgt mittels photometrischer Tests (Bergmeyer, 1983: "Methods of Enzymatic Analysis", S. 168-170 und 267-268).

Die Bestimmung von TMB/Gallussäure erfolgt chromatographisch mittels HPLC über eine Partisil ODS 3/5-Säule (Whatman Ltd. Springfield, GB).

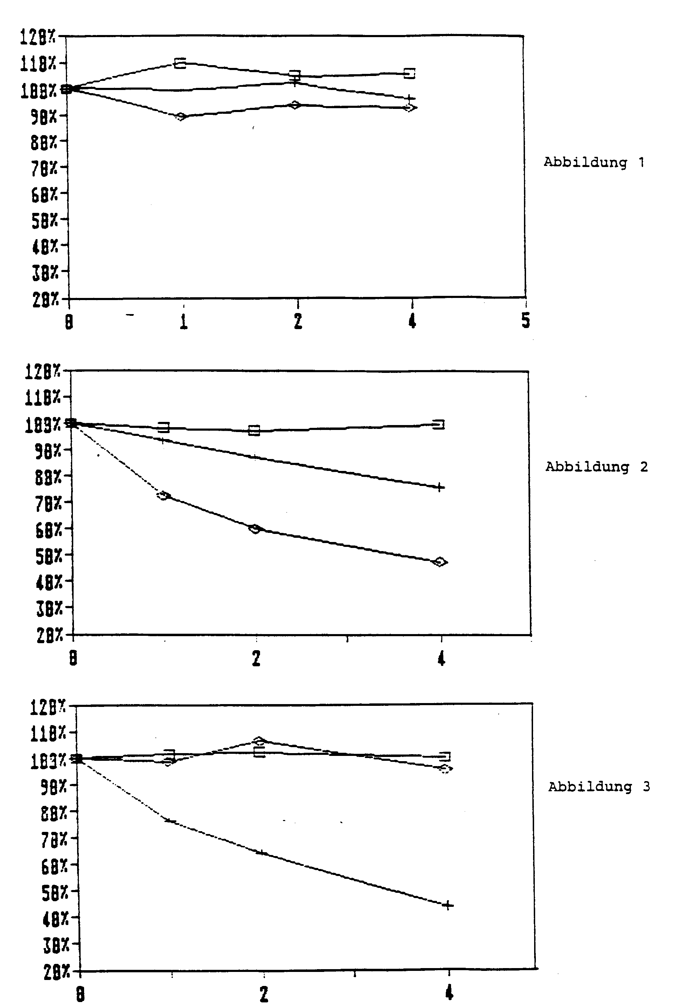

Der zeitliche Verlauf der Inhaltsstoffkonzentrationen in der jeweiligen Rezepturvariante I bis III ist in den Tabellen 1 bis 3 und Abbildungen 1 bis 3 dargestellt.

**Tabelle 1**

| (Masse I) | | | | |
|---|---|---|---|---|
| | Probennahmezeitpunkt (h) | | | |
| | 0 = 100% | 1 | 2 | 4 |
| Gallussäure | 100% | 99% | 102% | 95% |
| Tetramethylbenzidin | 100% | 109% | 104% | 113% |
| Cholesterinesterase | 100% | 84% | 78% | 89% |

**Tabelle 2**

| (Masse II) | | | | |
|---|---|---|---|---|
| | Probennahmezeitpunkt (h) | | | |
| | 0 = 100% | 1 | 2 | 4 |
| Gallussäure | 100% | 93% | 87% | 76% |
| Tetramethylbenzidin | 100% | 98% | 97% | 99% |
| Cholesterinesterase | 100% | 72% | 60% | 47% |

**Tabelle 3**

| (Masse III) | | | | |
|---|---|---|---|---|
| | Probennahmezeitpunkt (h) | | | |
| | 0 = 100% | 1 | 2 | 4 |
| Gallussäure | 100% | 76% | 64% | 44% |
| Tetramethylbenzidin | 100% | 101% | 102% | 100% |
| Cholesterinesterase | 100% | 99% | 106% | 95% |

### Ergebnis

In allen Rezepturen zeigt TMB keine signifikante Kinetik im Probennahmeintervall von 0 - 4h.

Tabelle 3 und Abbildung 3 zeigen, daß die Gallussäure im zeitlichen Verlauf zerstört wird.

Eine Verbesserung der Stabilität der Gallussäure kann durch Zugabe von Na₂H₂EDTA erreicht werden, da Spuren von Metallionen - insbesondere aus dem Rohstoff Celatom - komplexiert werden, bevor sie mit der Gallussäure wechselwirken.

Der Einsatz von Na₂H₂EDTA ist jedoch mit einer deutlichen Schädigung der magnesiumabhängigen Cholesterinesterase (CHE) verknüpft, wie aus der zeitlichen Abnahme der Aktivität der CHE. erkennbar ist (Masse II, Tab. 2 und Abb. 2).

Eine Stabilisierung von Gallussäure und CHE ergibt sich erst beim erfindungsgemäßen gleichzeitigen Zusatz von Mg²⁺-Ionen (K₂MgEDTA oder Mg²⁺Salze, z.B. MgCl₂, mit Na₂H₂EDTA; Masse I, Tab. 1 und Abb. 1).

## Patentansprüche

1. Verfahren zur Herstellung eines stabilisierten, aktiven Reagenzgemisches, welches ein kationenabhängiges Enzym enthält, das durch redoxaktive mehrwertige Kationen inhibiert wird, dadurch gekennzeichnet, daß man das kationenabhängige Enzym mit einem Salz eines Komplexbildners, das ein mehrwertiges nicht-redoxaktives Kation enthält oder
mit einem Komplexbildner oder ein Salz davon zusammen mit einem Salz eines mehrwertigen, nicht-redoxaktiven Kations
vermischt,
wobei die Menge des mehrwertigen nicht-redoxaktiven Kations größer oder gleich der Menge des Komplexbildners ist und die Menge des Komplexbildners größer als die Menge der anwesenden redoxaktiven Kationen ist.

2. Verfahren gemäß Anspruch 1, dadurch gekennzeichnet, daß dem Reagenzgemisch zusätzlich ein Bindemittel, Quellmittel und Kieselgur zugemischt werden.

3. Verfahren gemäß Anspruch 1 oder 2, dadurch gekennzeichnet, daß das nichtredoxaktive Kation ein Erdalkaliion ist.

4. Verfahren gemäß Anspruch 3, dadurch gekennzeichnet, daß das Erdalkaliion Magnesium ²⁺ oder Kalzium ²⁺ ist.

5. Verfahren gemäß einem der Ansprüche 1-4, dadurch gekennzeichnet, daß der Komplexbildner EDTA und das mehrwertige, nicht-redoxaktive Kation Magnesium ²⁺ ist.

6. Verfahren gemäß einem der Ansprüche 1-5, dadurch gekennzeichnet, daß das kationenabhängige Enzym Cholesterinesterase ist.

7. Aktives und stabilisiertes Reagenzgemisch enthaltend ein kationenabhängiges Enzym, das durch, redoxaktive mehrwertige Kationen inhibiert wird, dadurch gekennzeichnet, daß es zusätzlich ein Salz eines Komplexbildners, das ein mehrwertiges, nicht-redoxaktives Kation enthält oder einen Komplexbildner oder ein Salz davon und ein Salz eines mehrwertigen nicht-redoxaktiven Kations enthält, wobei die Menge des mehrwertigen nicht-redoxaktiven Kations größer oder gleich der Menge des Komplexbildners ist und die Menge des Komplexbildners größer als die Menge der anwesenden redoxaktiven Kationen ist.

8. Trockenes, stabilisiertes und aktives Trockenreagenzgemisch enthaltend ein kationenabhängiges Enzym, das durch redoxaktive mehrwertige Kationen inhibiert wird, dadurch gekennzeichnet, daß es zusätzlich ein Salz eines Komplexbildners, das ein mehrwertiges, nicht-redoxaktives Kation enthält oder einen Komplexbildner oder ein Salz davon und ein Salz eines mehrwertigen nicht-redoxaktiven Kations enthält, wobei die Menge des mehrwertigen nichtredoxaktiven Kations größer oder gleich der Menge des Komplexbildners ist und die Menge des Komplexbildners größer als die Menge der anwesenden redoxaktiven Kationen ist.

9. Trockenes Reagenzgemisch gemäß Anspruch 8, dadurch gekennzeichnet, daß es zusätzlich ein Bindemittel, Quellmittel und Kieselgur enthält.

10. Trockenes Reagenzgemisch gemäß Anspruch 9, dadurch gekennzeichnet, daß das mehrwertige, nicht-redoxaktive Kation ein Erdalkaliion ist.

11. Trockenes Reagenzgemisch gemäß Anspruch 10, dadurch gekennzeichnet, daß es als Komplexbildner EDTA und als Erdalkaliion Magnesium ²⁺ enthält.

12. Teststreifen zur Bestimmung eines Analyten in einer Probe, dadurch gekennzeichnet, daß er ein trockenes Reagenzgemisch gemäß einem der Anspruch 8-11 in Form eines auf einen festen Träger aufgetragenen Films enthält.

13. Verwendung eines Reagenzgemisches gemäß einem der Ansprüche 7-11 oder eines Teststreifens gemäß Anspruch 12 zur enzymatischen Bestimmung eines Analyten.

## Claims

1. Process for the preparation of a stabilised, active reagent mixture which contains a cation-dependent enzyme which is inhibited by redox-active polyvalent cations, characterised in that one mixes the cation-dependent enzyme with a salt of a complex former which contains a polyvalent non-redox-active cation or with a complex former or a salt thereof, together with a salt of a polyvalent, non-redox-active cation, whereby the amount of the polyvalent, non-redox-active cation is greater than or equal to the amount of the complex former and the amount of the complex former is greater than the amount of the redox-active cation present.

2. Process according to claim 1, characterised in that a binding agent, swelling agent and kieselguhr are additionally admixed with the reagent mixture.

3. Process according to claim 1 or 2, characterised in that the non-redox-active cation is an alkaline earth metal ion.

4. Process according to claim 3, characterised in that the alkaline earth metal ion is magnesium ²⁺ or calcium ²⁺.

5. Process according to one of claims 1 - 4, characterised in that the complex former is EDTA and the polyvalent, non-redox-active cation is magnesium ²⁺.

6. Process according to one of claims 1 - 5, characterised in that the cation-dependent enzyme is cholesterol esterase.

7. Active and stabilised reagent mixture containing a cation-dependent enzyme which is inhibited by redox-active polyvalent cations, characterised in that it additionally contains a salt of a complex former which contains a polyvalent, non-redox-active cation or a complex former or a salt thereof and a salt of a polyvalent, non-redox-active cation, whereby the amount of the polyvalent, non-redox-active cation is greater than or equal to the amount of the complex former and the amount of the complex former is greater than the amount of the redox-active cation present.

8. Dry, stabilized and active dry reagent mixture containing a cation-dependent enzyme which is inhibited by redox-active polyvalent cations, characterised in that it additionally contains a salt of a complex former which contains a polyvalent, non-redox-active cation or a complex former or a salt thereof and a salt of a polyvalent, non-redox-active cation, whereby the amount of the polyvalent, non-redox-active cation is greater than or equal to the amount of the complex former and the amount of the complex former is greater than the amount of the redox-active cation present.

9. Dry reagent mixture according to claim 8, characterised in that it additionally contains a binding agent, swelling agent or kieselguhr.

10. Dry reagent mixture according to claim 9, characterised in that the polyvalent, non-redox-active cation is an alkaline earth metal ion.

11. Dry reagent mixture according to claim 10, characterised in that it contains EDTA as complex former and magnesium ²⁺ as alkaline earth metal ion.

12. Test strip for the determination of an analyte in a sample, characterised in that it contains a dry reagent mixture according to one of claims 8 - 11 in the form of a film applied to a solid carrier.

13. Use of a reagent mixture according to one of claims 7 - 11 or of a test strip according to claim 12 for the enzymatic determination of an analyte.

## Revendications

1. Procédé de préparation d'un mélange de réactifs actif, stabilisé, contenant une enzyme sensible aux cations, qui est inhibée par des cations à valences multiples ayant une activité redox, caractérisé en ce qu'on mélange l'enzyme sensible aux cations avec un sel d'un agent complexant, qui contient un cation à valences multiples n'ayant pas d'activité redox, ou avec un agent complexant ou un de ses sels, conjointement avec un sel d'un cation à valences multiples n'ayant pas d'activité redox, la quantité du cation à valences multiples n'ayant pas d'activité redox étant supérieure ou égale à la quantité de l'agent complexant et la quantité de l'agent complexant étant supérieure à celle des cations ayant une activité redox présents.

2. Procédé selon la revendication 1, caractérisé en ce que l'on ajoute en outre au mélange de réactifs un agent liant, un agent gonflant et de la terre d'infusoires.

3. Procédé selon la revendication 1 ou 2, caractérisé en ce que le cation n'ayant pas d'activité redox est un ion d'un élément alcalino-terreux.

4. Procédé selon la revendication 3, caractérisé en ce que l'ion d'un élément alcalino-terreux est le magnésium²⁺ ou le calcium²⁺.

5. Procédé selon l'une quelconque des revendications 1-4, caractérisé en ce que l'agent complexant est l'EDTA et le cation à valences multiples n'ayant pas d'activité redox est le magnésium²⁺.

6. Procédé selon l'une quelconque des revendications 1-5, caractérisé en ce que l'enzyme sensible aux cations est la cholestérinestérase.

7. Mélange de réactifs actif et stabilisé, contenant une enzyme sensible aux cations, qui est inhibée par des cations à valences multiples ayant une activité redox, caractérisé en ce qu'il contient en outre un agent complexant qui contient un cation à valences multiples n'ayant pas d'activité redox, ou un agent complexant ou un de ses sels et un sel d'un cation à valences multiples n'ayant pas d'activité redox, la quantité du cation à valences multiples n'ayant pas d'activité redox étant supérieure ou égale à la quantité de l'agent complexant et la quantité de l'agent complexant étant supérieure à celle des cations ayant une activité redox présents.

8. Mélange de réactifs secs actif et stabilisé, sec, contenant une enzyme sensible aux cations, qui est inhibée par des cations à valences multiples ayant une activité redox, caractérisé en ce qu'il contient en outre un sel d'un agent complexant qui contient un cation à valences multiples n'ayant pas d'activité redox, ou un agent complexant ou un de ses sels et un sel d'un cation à valences multiples n'ayant pas d'activité redox, la quantité du cation à valences multiples n'ayant pas d'activité redox étant supérieure ou égale à la quantité de l'agent complexant et la quantité de l'agent complexant étant supérieure à celle des cations ayant une activité redox présents.

9. Mélange de réactifs sec selon la revendication 8, caractérisé en ce qu'il contient en outre un agent liant, un agent gonflant et de la terre d'infusoires.

10. Mélange de réactifs sec selon la revendication 9, caractérisé en ce que le cation à valences multiples n'ayant pas d'activité redox est un ion d'un élément alcalino-terreux.

11. Mélange de réactifs sec selon la revendication 10, caractérisé en ce qu'il contient, en tant qu'agent complexant, de l'EDTA et en tant que cation à valences multiples n'ayant pas d'activité redox, du magnésium²⁺.

12. Bande de test pour la détermination d'un analyte dans un échantillon, caractérisé en ce qu'elle contient un mélange de réactifs sec selon l'une quelconque des revendications 8-11 sous la forme d'un film fixé sur un support solide.

13. Utilisation d'un mélange de réactifs selon l'une quelconque des revendications 7-11 ou d'une bande de test selon la revendication 12, pour la détermination enzymatique d'un analyte.
